# EUROPEAN PATENT APPLICATION

(11) **EP 1 258 190 A1**
(43) Date of publication of application: **20.11.2002**
(21) Application number: 01112327.0
(22) Date of filing: 19.05.2001
(51) Int. Cl.: A01M 1/20, A61L 9/03, A61L 9/12

(54) **Unit for dispensing a volatile liquid**

(71) Applicant: Givaudan SA, 1214 Vernier (CH)
(72) Inventor: Brown, Colin William, Egham, Surrey TW20 8LP (GB); Hart, Gerald Leslie, Surbiton, Surrey KT5 8BD (GB); Naish, Guy, Narborough, Leicestershire LE9 5DD (GB); Gohil, Kishen, New Malden, Surrey KT3 6HB (GB)
(74) Representative: Patentanwälte Schaad, Balass, Menzl & Partner AG

(57) **Abstract**

The invention relates to a unit for dispensing a volatile liquid contained in a reservoir (10). The unit comprises a capillary medium (13) suitable for drawing the liquid from the reservoir, a heating element (2) arranged near a top end of the capillary medium (13) for evaporation of the liquid and an air stream generator (22) for dispersing the vapour.

## Description

The invention relates to a unit for dispensing a volatile liquid according to claim 1.

Plug-in air freshener or insecticide units are known that operate using a heater device which heats a capillary medium fed from a liquid reservoir. Such units for evaporation of a volatile liquid are described in US patents nos. 5,647,053, 5,290,546, 5,038,394 and 5,095,647 and in EP-A 0962132.

For example, the wick is formed in the shape of a rod that draws liquid from a refill container up and into a cylindrical gap formed at the center of a heater coil, through which the wick rod passes. Heat from the coil warms the rod, causing evaporation of the liquid, which is replenished through capillary action up the porous rod.

In all cases these units rely on the air motion resulting from the heat rising from the unit and the natural dispersion properties of the volatile liquid to disperse the vapour within a defined area such as a room.

As the vapour is distributed by convection only, a high intensity of fragrance in the surrounding air is hard to achieve. The intensity can be controlled by varying the temperature only. Therefore, to achieve a given intensity, the fragrance has to be treated with a given temperature which will not always be the temperature where the different notes of the fragrance develop in an optimum way. Further, these devices have a long reaction time after switching the heating on which makes them unsuitable for applications where fragrance has to be released on a time scale of minutes.

Another known way of dispensing a volatile material is to pass a stream of air over a volatile material, which may be in solid or liquid form. Such devices utilise an electric fan to introduce the flow of air over the volatile, as is taught in US patents nos. 5,547,616, 5,370,829, 5,147,582, 4,840,770 and 4,743,406.

In US 4,370,300 an evaporator is immersed in an aromatic odorant and a fan is utilised to control the evaporation rate of the ordorant in response to the temperature and illumination intensity in the room.

These devices rely on an air stream for evaporation of the liquid. The evaporation rate depends very much on the volatility of the liquid at the temperature of the surroundings. Often, the liquid is even cooled by the air stream which further reduces the volatility. A high fragrance intensity is in most cases not achievable.

It is therefore an object of the invention to propose a unit for dispensing a volatile liquid which overcomes the problems mentioned above. The evaporation and dispersion efficiency shall be increased.

This object is achieved by a unit for dispensing a volatile liquid as claimed in claim 1. Beneficial embodiments of the invention are described in the dependent claims, the description and the drawings.

The invention combines the use of a heater unit for evaporation of a volatile liquid with the use of an air stream generator to expel and distribute the vapour into the surrounding air. The heating element evaporates liquid from the top of the capillary medium from which it enters a preferably lateral air flow. Due to capillary action, further material is drawn into the capillary medium and to the heater.

The invention has several advantages. The dispersion of vapour is enhanced. The evaporated liquid is distributed efficiently by the air stream such that a given odour intensity can be achieved. The evaporated liquid is carried out of the device and does not form a liquid film on or inside the device when condesing. The optimum temperature for evaporation of the liquid and development of the odour can be chosen, as the invention uses an air stream for distribution of the vapour instead of relying on convection. The invention allows to switch an odour on or off with short reaction times.

In one embodiment of the invention the capillary medium and the heating element are arranged in a housing which is closed except for at least one air vent. The distribution of fragrance can be controlled precisely by adjusting the generated air stream and the temperature of the heating element. If both air stream generator and heating element are switched off, basically no passive distribution of liquid will occur.

Preferably the heating element comprises a ring-shaped heated member, e.g. a heating coil, arranged around the top end of the capillary medium, which is preferably a rod-shaped wick. Thereby the top end of the capillary medium is heated uniformly resulting in a good liquid transfer. In a further preferred embodiment, the heated member is used for holding the top end of the capillary medium in a fixed position.

The air stream generator, e.g. a small electric fan, can be arranged in different ways. In one embodiment it blows air and vapour from the heater unit and expels it trough a vent. In another embodiment it is arranged such that it sucks air and vapour from the heater unit trough a vent to the outside. In both cases it may be arranged inside or outside a housing of the device. The fan is arranged such that the air flow creates blows across the top of the heater unit, where the vapour is emitted, and out into the room.

The heater unit and the air stream generator are powered by electric means. In a preferred embodiment, both elements can be independently operated, preferably independently controlled, which allows for adjustment of the optimum temperature and air flow for a given fragrance and desired intensity. The heater unit and/or the air stream generator are powered by means of a battery inserted in the unit or from the domestic mains power supply. In the latter case the unit comprises electrical contact means for connecting the unit to the domestic mains power supply. Preferably, the contact means can be varied to connect to a plurality of plug sockets. Thus the device can be used in a plurality of countries, e.g. when travelling.

### Brief description of the drawings:

- Fig. 1: shows a schematic view of an inventive dispens-ing unit;
- Fig. 2a: shows an example for a circuit diagram for pow-ering the electric parts of the dispensing unit.

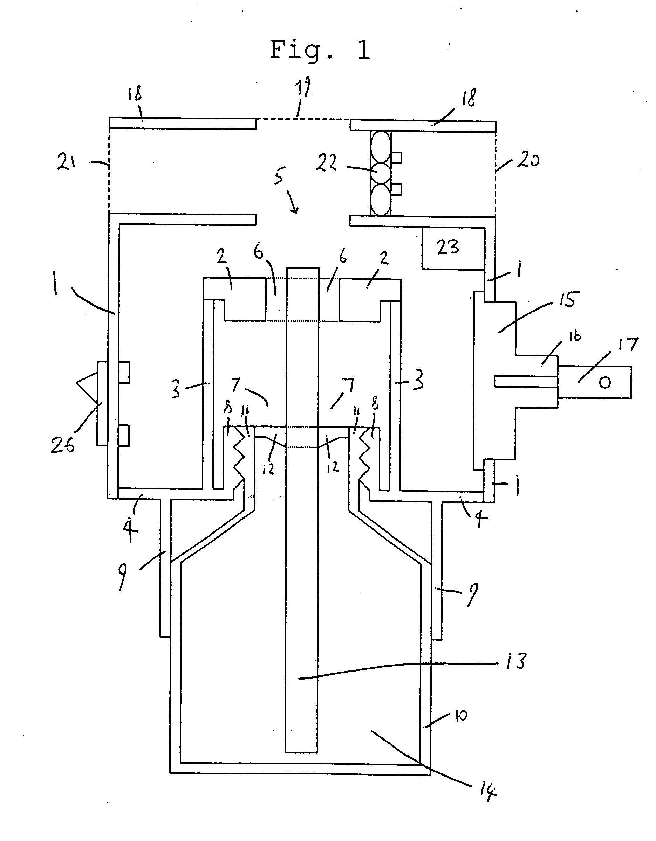

Fig. 1 shows an example for an inventive dispensing unit. The unit comprises a housing or a body 1 enclosing a ring-shaped heater unit 2. The heater is mounted on support columns 3 that are molded as part of the bottom portion 4 of the body, which is attached to the main body 1 as a separate piece. The top of the body 1 has an opening 5 corresponding to the top of the central hole 6 of the ring heater unit 2. The bottom portion of the body 4 has a similar opening 7 corresponding to the bottom of the central hole 6 of the ring heater unit 2. This opening 7 is surrounded by a threaded portion 8 and a flange 9 that is designed to accept and partly cover a refill bottle 10. The refill bottle 10 has a suitably threaded neck portion 11 that screws into the threaded portion 8 of the bottom potion of the body 4. The refill bottle 10 also has a neck insert 12 that tightly holds a rigid, porous wick or rod 13, which is immersed in the liquid 14, contained in the refill bottle 10. The rod 13 is of sufficient length that when the refill bottle 10 is screwed into the corresponding orifice 8, the rod 13 passes through the center of the ring heater 2. Built into one side of the unit body 1 is a circular portion 15 that can rotate about 90 degrees. Inserted into a central extension 16 of this circular portion 15 are electrical plug blades or pins 17 appropriate to the country in which the unit is to be used. At the top of the main body is a cap 18 with a top orifice 19 corresponding to the opening 5. The cap 18 also has two vented areas 20, 21 on opposite sides of the cap 18. Mounted inside the cap 18 is a small electrical blower fan 22, mounted such that the flow of air is perpendicular to the line defined by the rod 13. Connected to the fan 22 is a step-down transformer 23 that converts the alternating current mains supply to the 12-volt direct current supply required to power the fan.

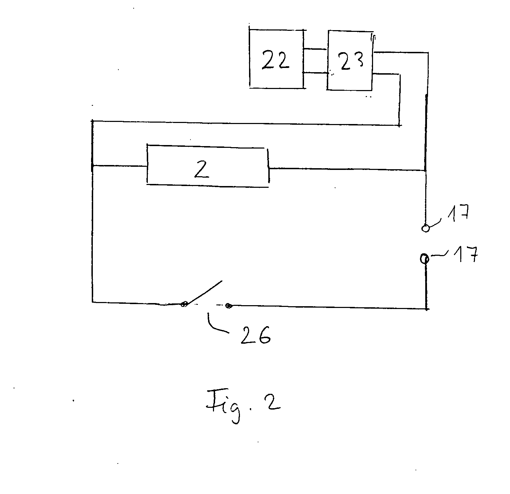

Fig. 2 shows a basic circuit diagram. The ring heater unit 2 is directly connected to the plug blades or pins 17 where the electrical circuit can be interrupted by operation of a switch 26. The fan 22 is connected to the step-down transformer 23. The step-down transformer 23 can be switched on and off by the switch 26. An optional addition, not shown in Fig. 2 in the form of a light that is activated when the switch 26 is turned to the on position to give a visual cue that the unit is activated. Switch 26 may also be an adjustable resistor which allows for controlling the temperature and air flow by controlling the electric currents respectively voltages.

In the operation of the device, the refill bottle is inserted into the threaded orifice of the main unit. It is envisaged that the refill bottle is supplied with a protective cover that screws over the porous rod and is discarded prior to use. Once the refill is inserted, the unit is plugged into a domestic power socket. This, it is envisaged that variations of the unit could be constructed with electrical pin of blade layouts corresponding to all the different power systems found in the domestic environment depending on the country of origin. It is also envisaged that variations of the electrical circuitry can be designed to run on any domestic voltage supply, i.e. 110V-240V, depending on the country of origin. Once plugged in, the unit can be independently activated by means of the built-in switch. On activation, the heater warms up, warming the wick rod and resulting in rapid evaporation of volatile liquid from the rod. Liquid is replenished from the refill bottle via capillary action drawing liquid up the porous rod. The resultant vapour then rises through the orifice in the main body of the unit and into the airflow of the fan. On activation of the switch, the fan is also turned on, drawing air through the vent at the back of the unit and expelling it along with the volatilized liquid through the front vent and out into the room. The unit also has a top orifice so that heated vapour can still escape through thermal convection if required. It is envisaged that the fan could be mounted adjacent to the front vent, operating in such a way that it sucks air across the top of the heater unit rather than as shown where it blows air across.

## Claims

1. Unit for dispensing a volatile liquid contained in a reservoir comprising a capillary medium (13) suitable for drawing the liquid from the reservoir, a heating element (2) arranged near a top end of the capillary medium (13) for evaporation of the liquid and a air stream generator (22) for dispersing the vapour.

2. Unit according to claim 1, wherein the heating element (2) comprises a ring-shaped heated member arranged around the top end of the capillary medium (13).

3. Unit according to claim 2, wherein the ring-shaped heated member holds the top end of the capillary medium (13).

4. Unit according to claim 1, 2 or 3, wherein the capillary medium (13) comprises at least one elongate rod or wick, preferably made of a porous material.

5. Unit according to one of the preceding claims, wherein the air stream generator (22) is arranged such that it blows air and vapour from the heating element and expels it trough a vent (20, 21).

6. Unit according to one of the claims 1 to 4, wherein the air stream generator (22) is arranged such that it sucks air and vapour from the heating element (2) and expels it trough a vent (20, 21).

7. Unit according to one of the preceding claims, wherein the heating element (2) and the air stream generator (22) are powered by electric means.

8. Unit according to claim 7, wherein the heating element (2) and the air stream generator (22) can be operated independent of each other.

9. Unit according to claim 7 or 8, wherein the heating element (2) and/or the air stream generator (22) are powered by means of a battery inserted in the unit.

10. Unit according to one of claims 7 to 9, wherein the heating element (2) and/or the air stream generator (22) are powered from the domestic mains power supply and wherein the unit comprises electrical contact means for connecting the unit to the domestic mains power supply.

11. Unit according to claim 10, wherein the electrical contact means comprise plug pins (17) and/or blades which can be varied to connect to a plurality of plug sockets.

12. Unit according to one of the preceding claims, wherein the reservoir is a refill bottle (10).

13. Unit according to one of the preceding claims, wherein the capillary medium and the heating element (2) are arranged in a housing (1) which comprises at least one air vent (20, 21).
